# EUROPÄISCHE PATENTANMELDUNG

(11) **EP 4 137 087 A1**
(43) Veröffentlichungstag der Anmeldung: **22.02.2023**
(21) Anmeldenummer: 21020417.8
(22) Anmeldetag: 16.08.2021
(51) Int. Cl.: A61B 34/10

(54) **ROBOTISCH GESTEUERTE FRÄSANWENDUNG IN DER WIRBELSÄULENCHIRURGIE**

(71) Anmelder: Hess, Michael, 85521 Riemerling (DE); Hess, Stefan, 20251 Hamburg (DE)
(72) Erfinder: Hess, Michael, 85521 Riemerling (DE); Hess, Stefan, 20251 Hamburg (DE)

(57) **Zusammenfassung**

Computergestütztes Verfahren zur Vorbereitung eines Eingriffs an der Wirbelsäule eines Patienten,mit den folgenden Schritten:
- Empfangen eines Patientendatensatzes beinhaltend Bilddaten des zu operierenden Operationsfeldes
- Identifizierung des Nervenaustrittskanals oder Austrittsloch des Spinalnervs sowie der Körperoberfläche mittels des empfangenen Bilddatensatzes
- Ermittlung eines Zugangsbereiches möglicher Zugänge zum Nervenaustrittskanal / Austrittsloch des Spinalnervs
- Visuelle Darstellung des ermittelten Zugangsbereiches.

## Beschreibung

### Bezeichnung der Erfindung

Durch künstliche Intelligenz robotisch gesteuerte Fräsanwendung in der Wirbelsäulenchirurgie.

### Angabe des technischen Gebiets, zu dem die Erfindung gehört

Ein Computerprogramm und technisches System zur Computer-gesteuerten robotisch durchgeführten Fräsanwendung in der Wirbelsäulenchirurgie; eine Methode zum Anlernen eines technischen Systems zum Identifizieren von Operationsparametern und Solllageinformationen in der Wirbelsäulenchirurgie.

### Stand der Technik

Endoskopische Operationen an der Wirbelsäule werden nach derzeitigem Stand manuell vorgenommen. Der Eintrittspunkt am Körper des Patienten befindet sich bei dem transforaminalen endoskopischen Zugang (Brust- und Lendenwirbelsäule) nicht über dem OP-Gebiet, sondern weit seitlich am Rumpf des Patienten. Dieser Punkt wird manuell bestimmt unter Zuhilfenahme von Durchleuchtung. Der Arzt führt die Zugangsinstrumente, in der Regel Dilatoren sowie Bohrer oder Fräsen mit der Hand an das zu operierende Bewegungssegment und entfernt dort Knochen- oder knochenähnliches Gewebe, um den operativen Zugang zum Spinalkanal herzustellen. Die Instrumente, in der Regel Bohrer oder Fräsen, werden dabei nach dem derzeitigen Stand der Technik mit der Hand geführt, um eine haptische Wahrnehmung (Gefühl) zu haben. Haptische Wahrnehmung ist wichtig um Druckstärke, Drehgeschwindigkeit, Drehmoment, Drehrichtung, und Druckdauer bestimmen zu können.

### Das der Erfindung zugrunde liegende Problem

Endoskopische Operationen an der Wirbelsäule werden durch den Operateur überwiegend manuell durchgeführt. Der Erfolg der Operationen ist damit abhängig von der Erfahrung und Kompetenz des Operateurs und Sicherheit und Erfolg des Eingriffs unterliegen Schwankungen. Wesentliche Erfolgsfaktoren des Eingriffs sind
- Das Auffinden des korrekten Eintrittspunktes an der Körperoberfläche: Der Hautschnitt ist nur einen Zentimeter groß, der optimale Eintrittspunkt dafür liegt in einem mutmaßlich 2-3 cm² großen Areal. Das operative Zielgebiet ist je nach betroffenem Bewegungssegment und Körpervolumen des Patienten 12-18 cm entfernt. Eine wesentliche Manipulation der Instrumente ist nicht möglich, Zugangswinkel und -weg können nicht mehr verändert werden. Es muss ausgeschlossen werden, dass der Zugang durch den Beckenknochen versperrt ist und dass keine Nerven oder andere wichtige anatomische Strukturen (Gefäße, Nieren), welche im Zugangsweg liegen, verletzt werden. Erst dann beginnt der operative Eingriff. Das Auffinden des korrekten Eintrittspunktes an der Körperoberfläche (Solllage) wird anhand von verschiedenen Pateientendaten durch den Operateur durchgeführt und ist daher von der Erfahrung und Kompetenz des Operateurs abhängig und unterliegt damit Schwankungen.
- Die präzise Übertragung der Einwirkung des Instruments auf Knochen- oder ähnliches Gewebe an der Wirbelsäule: Aufgrund der Notwendigkeit haptischer Wahrnehmung bei der Führung des Instruments ist die Sicherheit des Eingriffs abhängig von der Erfahrung und Kompetenz des Operateurs und unterliegt damit Schwankungen. Zu nennen sind hier insbesondere die Beibehaltung der Trajektorie des Zugangsweges im dreidimensionalen Raum, das Verhindern der Ablenkung der Fräse oder des mit fräsender Wirkung benutzen Bohrers (Parallelverschiebung nach kopfwärts in Richtung des Spinalnervs) und das Vermeiden eines plötzlichen Widerstandsverlustes (loss of resistance) und plötzlichem unkontrolliertem Vordringen des Instrumentes.

### Problemlösung und damit erreichte Vorteile

Die Sicherheit und der Erfolg des Eingriffs sollen durch folgende Lösungen erhöht und verstetigt werden:
- Verwendung von speziell entwickelten Fräsen mit patentierter Spitzenausformung (Fräskopf). Dieser trägt Knochen- oder ähnliches Gewebe effektiv ab unter Schonung der an der Wirbelsäule gelegenen Nerven; unser Patent Nr. 10 2016 115 634. [Dies ist nicht Gegenstand dieser Erfindung / Patentanmeldung.]
- Einbindung des oben genannten Fräskopfes oder mit fräsender Wirkung eingesetzten Bohrer in ein zu entwickelndes Computerprogramm und technisches System zur Computer-gesteuerten robotisch durchgeführten Fräsanwendung in der Wirbelsäulenchirurgie - dieses zu entwickelnde Computerprogramm und technisches System ist Gegenstand dieser Erfindung / Patentanmeldung. Die Erfindung benutzt einen Körperdatensatz, welcher neben den Diagnosedaten weitere Daten umfasst, wie beispielsweise die Körpermaße (Größe, Gewicht, Taillenumfang, u.a.) sowie Bilddaten. Hierzu soll eine Methode zum Anlernen eines Systems zum Identifizieren von Operationsparametern und Solllageinformationen in der Wirbelsäulenchirurgie entwickelt werden.

Durch die Methode soll eine präzise Übertragung der Einwirkung des Instruments auf Knochen- oder ähnliches Gewebe an der Wirbelsäule sichergestellt werden, um die Sicherheit des Eingriffs zu erhöhen. Hierfür ist notwendig, dass die Erfahrung und Kompetenz des Operateurs automatisiert wird, so dass die Operation nicht mehr manuell durch den Operateur, sondern automatisiert geführt wird. Dabei soll dem Operateur nur die Überwachung der Operation obliegen, die Durchführung erfolgt automatisch.

Zur automatischen Durchführung gehört ebenso die Ermittlung der Sollageinformation für den bestmöglichen operativen Zugang zum Spinalkanal.

Die Automatisierung erfordert Software (diese umfasst zumindest die Erfahrung und Kompetenz des Operateurs und Hardware (Instrument, welches durch die Software gesteuert wird).

### Erläuterung der Erfindung anhand eines Ausführungsbeispiels

Ein Bandscheibenvorfall (oder eine Stenose des Nervenaustrittsloches) ist der Zielpunkt.

Dieser variiert von Patient zu Patient. Der Zugang zum Zielpunkt wird beeinflusst von der Segmenthöhe (5 Bewegungssegmente L1-S1 an der Lendenwirbelsäule), von der Ausformung und Größe der Beckenknochen und der Lage und Größe der zu entfernenden krankhaften Veränderung (Vorfall seitlich, im Nervenaustrittsloch oder mittig), sowie Körpergröße, Körpergewicht und Body-Mass Index des Patienten.

Der Körperdatensatz enthält die oben genannten Informationen und weitere biometrische Angaben des Patienten beispielsweise in Form eines MRT-Bilddatensatzes aus einem präoperativen Patientenscan oder einer Patientendatenbank mit patienteneigenen oder standardisierten bzw. gemittelten Patientendaten. Diese Daten können beliebig kombiniert werden.

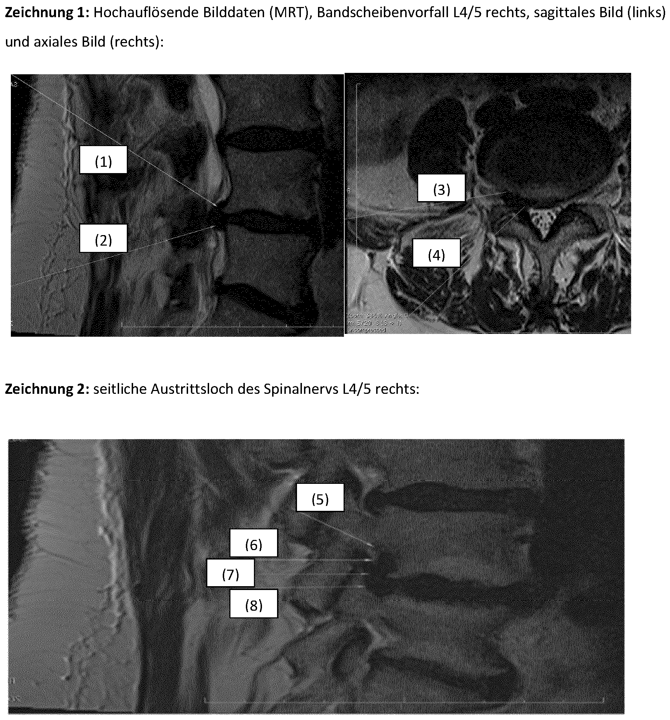

Im Folgenden wird auf die **Zeichnungen 1 und 2** Bezug genommen. Beschreibung der Zeichnungen:
- **Zeichnung 1:** Hochauflösende Bilddaten (MRT), Bandscheibenvorfall L4/5 rechts, sagittales Bild (links) und axiales Bild (rechts)
- **Zeichnung 2:** seitliche Austrittsloch des Spinalnervs L4/5 rechts

Die Linien (1) und (2) in **Zeichnung 1** sollen die Mantelfläche eines Kegels darstellen, dessen Spitze der Zielpunkt ist.

Die Grundfläche des Kegels in **Zeichnung 1** liegt auf der Hautoberfläche. Jeder Punkt innerhalb dieser Grundfläche stellt einen möglichen Eintrittspunkt für den OP-Zugang dar, der 1cm lang ist (Eintrittspunkt als kreisrunde Fläche mit 1 cm Durchmesser).

Problem: Einige dieser möglichen Zugangswege in **Zeichnung 1** gehen durch das Gelenk (violette Linie) (4) oder verletzen den Spinalnerv (grüne Linie) (3). Andere sind nicht möglich, weil dort der Beckenkamm ist (nicht auf den Bildern zu sehen). Das schränkt die Möglichkeiten erheblich ein.

Von den verbliebenen Optionen gibt es bessere, um den Vorfall in seiner ganzen Ausdehnung zu erreichen und schlechtere, die dies nicht oder nur unter größerem Aufwand (Dauer, Risiko) ermöglichen.

**Zeichnung 2** zeigt das seitliche Austrittsloch des Spinalnervs L4/5 rechts. Dieser befindet sich oben (grüner Pfeil) (5). Alle anderen Pfeile markieren den Bandscheibenvorfall bzw. die Einengung des Nervenaustrittsloches (6) bis (8).

Das zu entwickelnde technische System bestehend aus einem Computerprogramm und Hardware beinhaltet eine Ermittlungseinheit. Diese simuliert anhand des Körperdatensatzes mögliche Lageinformationen z.B. Lagepunkte, Lagewinkel und Durchmesser für den operativen Eingriff von außen im Hinblick auf Zugang zum Spinalkanal unter Berücksichtigung von zumindest der Entfernung zum Wirbelsäulenbereich (Bandscheibenvorfall), Knochen- oder knochenähnlichen Gewebe an der Wirbelsäule, sonstiges Gewebe und Organe zwischen dem Wirbelsäulenbereich und dem Lagepunkt.

Die Ermittlungseinheit ermittelt anhand der Simulation die Solllageinformation (optimaler Lagepunkt, Lagewinkel und Durchmesser für den operativen Eingriff von außen auf Basis eines lernenden Algorithmus unter Berücksichtigung der Entfernung zum Wirbelsäulenbereich (Bandscheibenvorfall), Knochen- oder knochenähnliches Gewebe an der Wirbelsäule, sonstiges Gewebe und Organe zwischen dem Wirbelsäulenbereich und dem Lagepunkt. Der Algorithmus basiert unter anderem auf einem gewichteten Scoring System und zu definierenden Bedingungen. Der Algorithmus umfasst unter anderem die Segmentation der Bilddaten und erkennt über tiefes lernen (deep learning), bzw. maschinenlernen die einzelnen Segmente bzw. Körperteile.

Nach Festlegung der Sollageinformation (optimaler Lagepunkt, Lagewinkel und Durchmesser) berechnet das zu entwickelnde System die Fläche und den 3D-Verlauf des Nervs, der geschont werden muss und - basierend auf dem Körperdatensatz und dem Durchmesser der benötigten Fräsen bzw. der mit fräsender Wirkung eingesetzten Bohrer ― wieviel vom Knochen im Bereich der hinteren Begrenzung des Nervenaustrittsloches (Spitze des rosafarbenen (7) und blauen Pfeiles (8) in **Zeichnung 2)** abgefräst werden muss.

Die Ermittlungseinheit stellt einer weiteren Einheit des zu entwickelnden Systems zusätzlich zum Körperdatensatz Werkzeugparameter zur Werkzeugwahl sowie Eingriffsvorgaben (Druckstärke, Drehgeschwindigkeit, Drehmoment, Drehrichtung, und Druckdauer) auf der Basis des Körperdatensatzes zur Verfügung.

Die weitere Einheit des technischen Systems beinhaltet roboterassistiertes Operationswerkzeug. Dieses ist dazu vorgesehen anhand der Solllageinformation eine definierte Sollposition anzufahren und anhand der Werkzeugparameter den Eingriff Computer-gesteuert durchzuführen.

## Patentansprüche

1. Computergestütztes Verfahren zur Vorbereitung eines Eingriffs an der Wirbelsäule eines Patienten, **gekennzeichnet durch** die folgenden Schritte
- Empfangen eines Patientendatensatzes beinhaltend Bilddaten des zu operierenden Operationsfeldes
- Identifizierung des *Nervenaustrittskanals oder Austrittsloch des Spinalnervs* sowie der Körperoberfläche mittels des empfangenen Bilddatensatzes
- Ermittlung eines Zugangsbereiches möglicher Zugänge zum *Nervenaustrittskanal* / *Austrittsloch des Spinalnervs*
- Visuelle Darstellung des ermittelten Zugangsbereiches.

2. Computergestütztes Verfahren gemäß dem vorangehenden Anspruch, wobei die Ermittlung des Zugangsbereiches vorzugsweise automatisch, anhand eines Algorithmus, der den Patientendatensatz auswertet, erfolgt, der Algorithmus umfassend
- eine Auswertung der Patientendaten bezüglich der Identifikation von erlaubten Bereichen und verbotenen Bereichen die zwischen dem Eintrittsbereich und dem Zielbereich liegen abhängig von definierten Bedingungen, insbesondere das Identifizieren des Nervenaustrittskanals oder Austrittslochs des Spinalnervs, Gelenken, Knochen wie Beckenkamm und Nerven wie Spinalnerv.
- Ermittlung des Zugangsbereiches möglicher Zugänge abhängig von den erlaubten und verbotenen Bereichen.
- Bestimmung eines optimalen Zugangs aus dem ermittelten Zugangsbereich, wobei der optimale Zugang den Eintrittspunkt auf der Haut, den Eintrittswinkel und die Entfernung zum Nervenaustrittskanal oder Austrittsloch beinhalten.

3. Computergestütztes Verfahren gemäß Anspruch 2, die Bedingungen weiter umfassend Entfernung des Körpereintritts zum Zielbereich und/oder Eintrittswinkel in den Körper und/oder Abstand zu verbotenen Bereichen.

4. Computergestütztes Verfahren gemäß Ansprüchen 2 oder 3, die Bedingungen umfassend gemittelte Vergleichsdaten von vorangehenden Patienten.

5. Computergestütztes Verfahren gemäß einem der vorangehenden Ansprüche, wobei der Zugangsbereich in Form eines Kegels berechnet und dargestellt wird.

6. Computergestütztes Verfahren gemäß Anspruch 5, wobei die Position der Spitze des Kegels der Position des identifizierten *Nervenaustrittskanals* / *Austrittsloch des Spinalnervs* entspricht.

7. Computergestütztes Verfahren gemäß einem der Ansprüche 5 und 2, wobei die Ermittlung des Zugangsbereichs das Berechnen und Darstellen der Schnittlinie des Kegels mit der Hautoberfläche beinhaltet, insbesondere wobei die Grundfläche des Kegels in etwa mit der Hautoberfläche deckungsgleich ist.

8. Computergestütztes Verfahren gemäß einem der vorangehenden Ansprüche, wobei das Ermitteln des Zugangsbereichs das Ausschließen von Zugangswegen umfasst die durch das Gelenk gehen oder den Spinalnerv verletzen.

9. Computergestütztes Verfahren wobei das Verfahren präoperativ durchgeführt wird.

10. System zur Vorbereitung und Durchführung eines Eingriffs an der Wirbelsäule eines Patienten beinhaltend einen Prozessor zur Durchführung des Verfahrens gemäß der Ansprüche 1-9.

11. System gemäß Anspruch 10 beinhaltend eine Anzeigevorrichtung wobei der Prozessor ausgelegt ist die visuelle Darstellung in der Anzeigevorrichtung anzuzeigen.

12. System gemäß den Ansprüchen 10 und 11 beinhaltend eine Robotikeinheit und/oder Werkzeuge zur Durchführung des Eingriffs an der Wirbelsäule, vorzugsweise wobei die Werkzeuge an der Robotikeinheit befestigbar sind.

13. System gemäß den Ansprüchen 10-12 beinhaltend eine Navigationseinheit, vorzugsweise mit Markern und Sensoreinheit zur Erfassung der Marker, zum Nachverfolgen der Wirbelsäule, der Robotikeinheit und der Werkzeuge.

14. System gemäß den Ansprüchen 10-13 wobei der Prozessor einen Speicher umfasst mit einer Datenbank mit Werkzeugdaten und der Prozessor ausgelegt ist aus dem ermittelten optimalen Zugangsbereich, den anatomischen Verhältnissen und den Werkzeugdaten eine geeignete Werkzeugauswahl und Eingriffsvorgaben (Druckstärke, Drehgeschwindigkeit, Drehmoment, Drehrichtung, und Druckdauer) zu treffen und die Robotikeinheit unter Zuhilfenahme der Navigationseinheit und des Werkzeugs derart anzusteuern um einen operativen Eingriff zur Erzeugung des Zugangs zum Nervenaustrittskanal oder Austrittsloch des Spinalnervs durchzuführen.

15. Computer-Programm-Produkt mit auf einem maschinenlesbaren Träger gespeicherten Programmcode zum Ausführen eines Verfahrens gemäß mindestens einem der Ansprüche 1-9, wenn das Programm-Produkt auf einem Computer ausgeführt wird.
